# EUROPEAN PATENT APPLICATION

(11) **EP 4 846 938 A1**
(43) Date of publication of application: **02.12.2026**
(21) Application number: 25744588.2
(22) Date of filing: 21.01.2025
(51) Int. Cl.: A61B 5/145, A61B 50/30

(54) **CASSETTE DEVICE, SENSOR ASSEMBLY PICKUP METHOD, AND ANALYTE MONITORING SYSTEM**

(30) Priority: 25.01.2024 CN 202410110470
(71) Applicant: SHENZHEN SISENSING CO., LTD., Shenzhen Guangdong 518110 (CN)
(72) Inventor: LI, Yunfeng, Shenzhen, Guangdong 518110 (CN)
(74) Representative: Vossius & Partner Patentanwälte Rechtsanwälte mbB
(86) International application number: PCT/CN2025/073765
(87) International publication number: WO 2025/157149

(57) **Abstract**

The disclosure describes a cartridge device, a pickup method of a sensor component, and an analyte monitoring system, the cartridge device includes a housing having an accommodating space, a support mechanism removably supporting the sensor component, and an assist mechanism, the support mechanism, the assist mechanism, and the sensor component supported by the support mechanism are located in the accommodating space, the support mechanism is tightly integrated with the sensor component to restrict movement of the sensor component, and the assist mechanism provides a force to the pickup device away from the support mechanism when the sensor component moves away from the support mechanism under the action of the pickup device coupled to the sensor component. According to the disclosure, there is provided a cartridge device, a pickup method of a sensor component, and an analyte monitoring system with high reliability in the pickup process of the sensor component.

## Description

### BACKGROUND OF INVENTION

### Field Of Invention

The disclosure relates to the field of medical devices, and particularly to a cartridge device, a pickup method of a sensor component, and an analyte monitoring system.

### Description of Related Art

Diabetes mellitus and its chronic complications have become one of the serious conditions affecting human health today. To delay and reduce the chronic complications of diabetes mellitus, strict glucose control is required; currently, continuous glucose monitoring systems (CGMS) for dynamically reflecting glucose fluctuations are widely used.

CGMS typically employs a subcutaneous sensor connected to an electronic component to monitor glucose concentration in body fluid; since the sensor must be implanted into the host during use, the probe must be sufficiently sterilized and disinfected prior to use for the health and safety of the host. Common sterilization methods for sensors include radiation sterilization (e.g., electron beam sterilization and gamma ray sterilization), if the electronic component and the sensor component are sterilized together by radiation, the circuitry of the electronic component may be damaged, therefore, techniques exist in which the sensor portion is sterilized separately, and the sensor component is assembled with the electronic component prior to use. In particular, the sensor component is typically contained, sterilized, and sealed in a separate container, prior to use, the sensor component in the container is picked up by an applicator containing the electronic component, the electronic component and sensor component are coupled into a complete sensor control device and removed from the container under the action of the applicator, and then the sensor control device is applied to the body surface of the user by the applicator. In order for the sensor control device applied to the user to remain on the user's body surface free of the applicator, the electronic component is typically removably arranged in the applicator, and the applicator does not exert excessive retention force on the electronic component.

However, during the process in which the electronic component and the sensor component are coupled into a sensor control device and then separated from the container, if the retention force exerted by the container on the sensor component is large, overcoming this force to release the sensor component from its retained state may cause the electronic component to be pulled by this force, resulting in displacement or even detachment from the applicator, thereby affecting the overall reliability of the monitoring kit.

### SUMMARY OF INVENTION

The disclosure has been made in view of the above-mentioned state of the art, and it is an object of the disclosure to provide a cartridge device, a pickup method for a sensor component, and an analyte monitoring system with high reliability in the pickup process of the sensor component.

To this end, a first aspect of the disclosure provides a cartridge device for retaining a sensor component, the cartridge device includes a housing having an accommodating space, a support mechanism removably supporting the sensor component, and an assist mechanism, the support mechanism, the assist mechanism, and the sensor component supported by the support mechanism are located in the accommodating space, the support mechanism is tightly integrated with the sensor component to restrict movement of the sensor component, and the assist mechanism provides a force to the pickup device away from the support mechanism when the sensor component moves away from the support mechanism under the action of the pickup device coupled to the sensor component.

In the cartridge device according to the first aspect of the disclosure, the cartridge device includes a support mechanism and an assist mechanism, the support mechanism is tightly integrated with the sensor component to enable the sensor component to be stably arranged in the cartridge device, and facilitate the alignment of the pickup device and the sensor component when the pickup device is subsequently used to pick up the sensor component; by means of the assist mechanism providing a force to the pickup device away from the support mechanism when the pickup device leaves the support mechanism after picking up the sensor component, the influence of the retention force exerted by the support mechanism on the sensor component upon the structural integrity of the pickup device itself may be reduced (i.e., the assisting force provided by the assist mechanism may partially offset the retention force of the support mechanism), thereby facilitating the smooth separation of the sensor component tightly integrated with the support mechanism from the support mechanism under the action of the pickup device and improving the reliability of the pickup device in picking up the sensor component. Thus, a cartridge device is provided that may stably retain the sensor component and enhance the reliability of the pickup device in picking up the sensor component.

Further, in the cartridge device according to the first aspect of the disclosure, optionally, the pickup device includes an applicator removably retaining an electronic component, the applicator is coupled to the cartridge device to pick up the sensor component such that the sensor component and the electronic component are coupled to form a patch component, the assist mechanism providing a force to the electronic component away from the support mechanism. In this case, after the applicator couples the electronic component and the sensor component to form the patch component, the force applied by the applicator to the electronic component in a direction away from the support mechanism is transmitted to the sensor component through the electronic component, conversely, when the sensor component disengages from the support mechanism, the retention force exerted by the support mechanism on the sensor component is transmitted to the electronic component via the sensor component, at this time, the electronic component, which is removably retained on the applicator, is simultaneously subjected to a force directed away from the support mechanism and a resistance force derived from the retention force of the support mechanism, the force provided by the assist mechanism to the electronic component may offset part of this resistance, thereby reducing the likelihood of undesirable displacement or even detachment of the electronic component from the applicator due to this resistance, thus improving the reliability of the applicator in picking up the sensor component from the cartridge device via the removably retained electronic component.

Further, in the cartridge device according to the first aspect of the disclosure, optionally, the assist mechanism includes a drive portion, during removal of the patch component from the support mechanism, the drive portion abuts a surface of the electronic component facing away from the applicator to provide a force to the electronic component away from the support mechanism. In such a case, when the drive portion provides a force to the electronic component away from the support mechanism to partially counteract the resistance of the support mechanism, the force may also help to press the electronic component against the applicator, further improving the stability of the electronic component retained in the applicator.

Further, in the cartridge device according to the first aspect of the disclosure, optionally, the drive portion includes an elastic element, the elastic element is in a compressed state when the applicator picks up the sensor component; during removal of the patch component from the support mechanism, an end portion of the drive portion contacts a surface of the electronic component facing away from the applicator to provide a force to the electronic component away from the support mechanism. In this case, the elastic element in a compressed state after pickup completion stores elastic potential energy, and during the separation of the patch component from the support mechanism, contact between the drive portion, including the elastic element and the surface of the electronic component enables the provision of a force to the electronic component away from the support mechanism using the elastic potential energy.

Further, in the cartridge device according to the first aspect of the disclosure, optionally, the surface of the electronic component facing away from the applicator is an adhesive surface, and a contact area of the drive portion with the adhesive surface accounts for not more than 10% of the total area of the adhesive surface. During use, the applicator generally applies the patch component to the body surface of the host; in this case, configuring the surface of the electronic component facing away from the applicator as an adhesive surface facilitates detachment of the electronic component from the applicator and retention on the host's body surface through adhesion to the host's body surface after application; configuring the contact area of the drive portion with the adhesive surface to account for not more than 10% of the total area of the adhesive surface allows reduction of the pulling force on the electronic component due to adhesion when the drive portion detaches from the adhesive surface, while effectively enabling force transmission between the assist mechanism and the electronic component, thereby facilitating stable retention of the electronic component in the applicator.

Further, in the cartridge device according to the first aspect of the disclosure, optionally, a surface of the electronic component facing away from the applicator includes a first region having adhesiveness, and a second region having a weaker adhesiveness than the first region, the first region is located at an outer periphery of the second region, the drive portion is in contact with the second region. In this case, configuring the surface of the electronic component facing away from the applicator to include a first region with adhesiveness facilitates detachment of the electronic component from the applicator and retention on the host's body surface through adhesion after application; additionally, arranging a second region with weaker adhesiveness and configuring the drive portion to contact the second region reduces the pulling force on the electronic component due to adhesion upon separation of the drive portion from the second region, while maintaining effective force transmission, thereby facilitating stable retention of the electronic component in the applicator.

Further, in the cartridge device according to the first aspect of the disclosure, optionally, an outer contour of a portion of the drive portion in contact with the electronic component is a curved surface. In this case, the contact area between the drive portion and the electronic component may be reduced on the basis of effectively achieving force transmission, so as to reduce the pulling force on the electronic component due to adhesion that may occur when the drive portion is separated from the electronic component, thereby allowing the stable retention of the electronic component in the applicator; additionally, compared to a sharp surface, forming the contact surface as a curved surface may also reduce potential damage to the electronic component when the drive portion applies a force to the electronic component.

Further, in the cartridge device according to the first aspect of the disclosure, optionally, further including a retaining portion for tightly retaining the sensor component, the retaining portion being a male-female mechanical mating structure respectively arranged on the support mechanism and the sensor component, the male-female mechanical mating structure being a clearance fit and/or an interference fit. In this case, the sensor component may be tightly integrated with the support mechanism by means of a male-female mechanical mating structure.

Further, in the cartridge device according to the first aspect of the disclosure, optionally, further including a limiting cover located in the accommodating space, the limit cover being arranged over the sensor component and having a limiting structure arranged around an outer periphery of at least part of members of the sensor component in a manner that does not contact the sensor component, and after the limiting cover leaving the accommodating space, the sensor component being in a pickable state. During transportation, the cartridge device may vibrate; in this case, the limiting cover having the limiting structure arranged around a portion of the outer periphery of the sensor component may further restrict undesirable displacement and/or rotation of the sensor component, maintaining the sensor component in a predetermined position to facilitate alignment when subsequently picked up by a pickup device; Compared with directly contacting the sensor component, arranging the limiting structure around the outer periphery of the sensor component without contact may reduce undesirable displacement of the sensor component caused by friction resulting from movement of the limiting structure.

Further, in the cartridge device according to the first aspect of the disclosure, optionally, further including a retention mechanism for retaining the limiting cover, the retention mechanism being configured to provide a predetermined retention force to the limiting cover, the retention force being greater than a gravitational force of the limiting cover. The cartridge device as a whole may vibrate or even be inverted during transportation or when the user opens the cartridge device; in this case, using the retention mechanism to retain the limiting cover may reduce the occurrence of unwanted movement of the limiting cover within the housing or even falling out of the housing.

Further, in the cartridge device according to the first aspect of the disclosure, optionally, further including an opening portion and a sealing portion, the accommodating space of the housing communicating with the exterior via the opening portion, the sealing portion being detachably arranged at the opening portion to seal the accommodating space. In this case, after sterilization, the accommodating space forms a sealed chamber, reducing contamination from the external environment; when use is required, removing the sealing portion from the opening portion places the interior members of the housing in an unsealed state for operation.

A second aspect of the disclosure provides a pickup method of a sensor component, including: tightly and removably retaining the sensor component using a support mechanism in a container; picking up the sensor component using a pickup device coupleable to the container, and during pickup, applying a force to the pickup device away from the support mechanism using an assist mechanism.

In the pickup method according to the second aspect of the disclosure, the sensor component is removably retained by tightly integrating the support mechanism in the cartridge device with the sensor component, enabling the sensor component to be stably arranged in the cartridge device and facilitating alignment between the sensor component and the pickup device during subsequent pickup operations; by means of the assist mechanism providing a force to the pickup device away from the support mechanism upon pickup of the sensor component bywhen the pickup device leaves the support mechanism after picking up the sensor component, the influence of the retention force exerted by the support mechanism on the sensor component upon the structural integrity of the pickup device itself may be reduced (i.e., the assisting force provided by the assist mechanism may partially offset the retention force of the support mechanism), thereby facilitating the smooth separation of the sensor component tightly integrated with the support mechanism from the support mechanism under the action of the pickup device and improving the reliability of the pickup device in picking up the sensor component. As a result, a high-reliability pickup method for the sensor component may be provided.

A third aspect of the disclosure provides an analyte monitoring system including: a sensor component, a cartridge device removably retaining the sensor component, an electronic component, and an applicator removably retaining the electronic component, the cartridge device including a support mechanism removably supporting the sensor component, and an assist mechanism, the support mechanism being tightly integrated with the sensor component to restrict movement of the sensor component, the applicator being coupled to the cartridge device to couple the electronic component with the sensor component to form a patch component, as the sensor component moving away from the support mechanism under the action of the electronic component and the applicator, the assist mechanism providing a force to the electronic component away from the support mechanism.

In the analyte monitoring system according to the third aspect of the disclosure, the cartridge device includes a support mechanism and an assist mechanism, the support mechanism is tightly integrated with the sensor component to enable the sensor component to be stably arranged in the cartridge device, and facilitate the alignment of the applicator and the sensor component when the applicator is subsequently used to pick up the sensor component; by means of the assist mechanism providing a force to the pickup device away from the support mechanism when the pickup device leaves the support mechanism after picking up the sensor component, the influence of the retention force exerted by the support mechanism on the sensor component upon the structural integrity of the pickup device itself may be reduced (i.e., the assisting force provided by the assist mechanism may partially offset the retention force of the support mechanism), reducing the likelihood of undesirable displacement or even detachment of the electronic component from the applicator due to such resistance, thereby facilitating the smooth separation of the sensor component tightly integrated with the support mechanism from the support mechanism under the action of the applicator and improving the reliability of the applicator in picking up the sensor component. Thus, an analyte monitoring system with high reliability in the sensor component pickup process may be provided.

Further, in the analyte monitoring system according to the third aspect of the disclosure, optionally, the sensor component includes a connecting base, and a sensor and a sharp object arranged on the connecting base, the electronic component includes an outer casing and a receiving portion for receiving the sensor component, the receiving portion including an accommodating portion and a through hole longitudinally penetrating the outer casing, when the electronic component is coupled to the sensor component, the connecting base is embedded in the accommodating portion and the sharp object is at least partially located within the through hole. In this case, the cartridge device may accommodate the sensor and the sharp object to be inserted into the host, enabling simultaneous sterilization, and by mounting both the sensor and the sharp object on the connecting base, they may be retained at a predetermined position, thus facilitating alignment during subsequent pickup operations; moreover, embedding the connecting base into the accommodating portion enhances the stability of the component coupling between the electronic component and the sensor component, and facilitates the applicator acting on the electronic component to carry the sensor component away from the support mechanism together during the pickup process.

Further, in the analyte monitoring system according to the third aspect of the disclosure, optionally, the applicator includes a first drive mechanism, a second drive mechanism, a proximal end close to the host and a distal end away from the host during operation, the first drive mechanism is configured to apply a force to the patch component in a manner toward the proximal end, the patch component is driven by the first drive mechanism toward the proximal end to place the sensor at least partially subcutaneously into the host via the sharp object, and after the sharp object reaches a predetermined position, the sharp object is driven by the second drive mechanism toward the distal end to withdraw the sharp object from the host. Thus, application of the patch component to the host and removal of the sharp object from the host may be achieved by the two drive mechanisms of the applicator.

According to the disclosure, it is possible to provide a cartridge device, a pickup method of a sensor component, and an analyte monitoring system that can improve the reliability of the pickup process of the sensor component.

### BRIEF DESCRIPTION OF THE DRAWINGS

The disclosure will now be explained in further detail, by way of example only, with reference to the accompanying drawings.
FIG. 1 is a diagram showing an application scenario of an analyte monitoring system according to an example of the disclosure.
FIG. 2A is a schematic view showing a first perspective view of a sensor component and an electronic component in accordance with an example of the disclosure.
FIG. 2B is a schematic view showing a second perspective view of a sensor component and an electronic component in accordance with an example of the disclosure.
FIG. 2C is a schematic view showing the coupling of a sensor component and an electronic component into a patch component in accordance with an example of the disclosure.
FIG. 3 is a schematic view showing a cartridge device according to an example of the disclosure.
FIG. 4 is a cross-sectional schematic view showing a cartridge device according to an example of the disclosure.
FIG. 5 is an exploded schematic view showing a cartridge device according to an example of the disclosure.
FIG. 6A is a schematic view showing a support mechanism according to an example of the disclosure.
FIG. 6B is a schematic view showing a sensor component supported by a support mechanism in accordance with an example of the disclosure.
FIG. 7A is a schematic view showing an assist mechanism according to an example of the disclosure in a natural state.
FIG. 7B is a schematic view showing an assist mechanism according to an example of the disclosure in a compressed state.
FIG. 8 is a schematic view showing a limiting cover positioned in a housing according to an example of the disclosure.
FIG. 9A is a first flowchart showing a pickup method according to an example of the disclosure.
FIG. 9B is a second flowchart showing a pickup method according to an example of the disclosure.
FIG. 10 is an exploded schematic view showing an applicator according to an example of the disclosure.

### DETAILED DESCRIPTION

Hereinafter, preferred embodiments of the disclosure will be described in detail with reference to the accompanying drawings. In the following description, the same reference numerals are assigned to the same components, and the repeated description thereof will be omitted. Additionally, the drawings are only a schematic view, and the proportions of the dimensions of the parts relative to each other, the shapes of the parts, etc., may be different from those in practice.

It should be noted that the terms "include" and "comprise", and any variations thereof, for example, a process, method, system, article, or apparatus that comprises or has a list of steps or elements in this disclosure are not necessarily limited to those steps or elements expressly listed, but may include or have other steps or elements not expressly listed or inherent to such process, method, article, or apparatus.

It should be noted that in this document, relative position and relative direction terms, such as "upper", "upward", "lower", "downward", "vertical direction", "left", "leftward", "left side", "toward the left", "right", "rightward", "right side", "toward the right", "lateral direction", "front", "forward", "rear", "rearward", "longitudinal direction", and the like, are with reference to a normal operating orientation and should not be considered limiting.

The disclosure is directed to a cartridge device that may be used to retain a sensor component, and a pickup device that may cooperate with the cartridge device to pick up the sensor component. With the cartridge device of the disclosure, the reliability of the pickup device in picking up the sensor component may be improved. In some examples, the pickup device may include a take-out device that removably retains a to-be-assembled component, and the take-out device may couple with the cartridge device to pick up the sensor component, thereby coupling the to-be-assembled component with the sensor component to form a target assembly. In some examples, the take-out device may be an applicator, and the to-be-assembled component may be an electronic component. That is, the pickup device may include an applicator that removably retains the electronic component. The sensor component may be removed by coupling the applicator with the cartridge device to couple the electronic component with the sensor component and form a patch component. At this point, the cartridge device may be part of an analyte monitoring system. In the disclosure, the cartridge device may also be referred to as a packaging box, packaging container, accommodating box, sterilization container, or sterilization box.

In this disclosure, the term "removably retained" means that a component retained by a retaining component may be detached from the retaining component under an external force.

The disclosure also relates to a pickup method for a sensor component. In the disclosure, the method for picking up a sensor component may be simply referred to as a pickup method, or may also be called an assembly method, an assembling method, etc.

The disclosure relates to an analyte monitoring system that may be used to monitor an analyte. In the disclosure, the analyte monitoring system may be briefly referred to as a monitoring system, or may also be referred to as an analyte detection system, an analyte measurement system, or a physiological parameter monitoring system. The analyte monitoring system may include a cartridge device accommodating a sensor component and an applicator accommodating an electronic component, wherein the applicator may couple with the cartridge device to assemble the electronic component and the sensor component into a patch component for picking up the sensor component, then the patch component is applied to a host, enabling acquisition of parameters related to the analyte and monitoring of the target analyte (e.g., continuous monitoring). In the disclosure, the patch component may also be referred to as a medical device, a continuous monitoring device, or an analyte monitoring device.

In the disclosure, an analyte may refer to a target substance to be analyzed. For example, the analyte may be one or more of glucose, acetylcholine, amylase, bilirubin, cholesterol, chorionic gonadotropin, creatine kinase, creatine, creatinine, DNA, fructosamine, glutamine, growth hormone, hormone, ketone body, lactate, oxygen, peroxide, prostate-specific antigen, prothrombin, RNA, thyroid-stimulating hormone, or troponin.

Hereinafter, the cartridge device involved in the disclosure will be described in detail with reference to the accompanying drawings. For ease of understanding, the cartridge device will be described in detail by way of example, in which the pickup device may include an applicator that removably retains an electronic component (i.e., where the cartridge device is part of an analyte monitoring system). It should be noted that the disclosure is not limited thereto, and the pickup device may alternatively be other devices configured to pick up the sensor component.

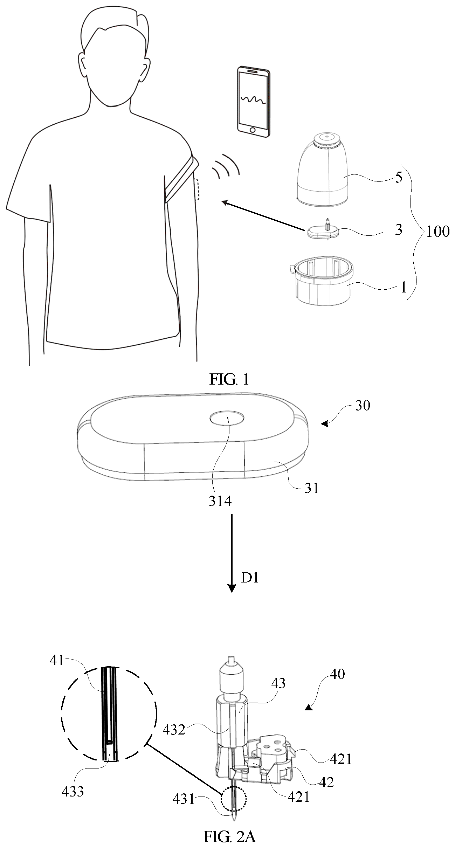

FIG. 1 is a diagram showing an application scenario of an analyte monitoring system 100 according to an example of the disclosure.

In the disclosure, the analyte monitoring system 100 may include a patch component 3, an applicator 5, and a cartridge device 1. The patch component 3 may be divided into two parts, with each part respectively accommodated in the applicator 5 and the cartridge device 1, the applicator 5 may remove the part of the members accommodated in the cartridge device 1 from the cartridge device 1 and assemble it into a complete patch component 3, the patch component 3 is then applied to the body surface of the host, thereby enabling monitoring of the target analyte via the patch component 3. Referring to FIG. 1, a patch component 3 may be placed on a body surface of a host.

In some examples, the patch component 3 may be divided into an electronic component 30 and a sensor component 40 (shown later). The electronic component 30 may be removably retained in the applicator 5, and the sensor component 40 may be removably retained to the cartridge device 1. That is, the electronic component 30 and the sensor component 40 may be coupled to form the patch component 3.

In some examples, the surface of the electronic component 30 facing away from the applicator 5 may be an adhesive surface. In this case, after the electronic component 30 is applied to the body surface of the host by the applicator 5, the adhesive surface may adhere to the skin of the host, and when the applicator 5 is removed from the body surface, the electronic component 30 may be separated from the applicator 5 by an adhesive force and retain on the body surface of the host.

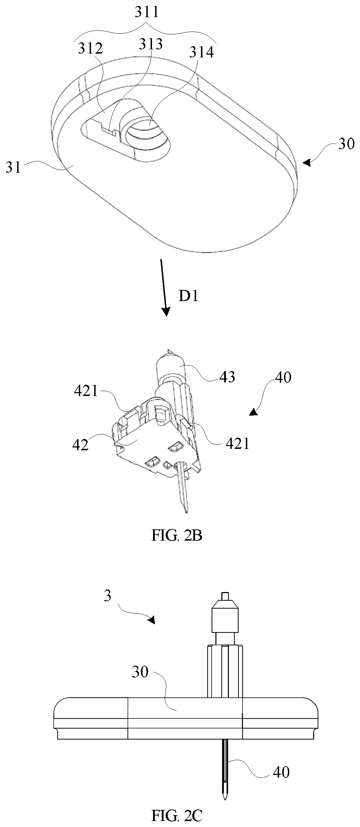

FIG. 2A is a schematic view showing a first perspective view of a sensor component 40 and an electronic component 30 in accordance with an example of the disclosure. FIG. 2B is a schematic view showing a second perspective view of the sensor component 40 and the electronic component 30 in accordance with an example of the disclosure. FIG. 2C is a schematic view showing the coupling of the sensor component 40 and the electronic component 30 to form the patch component 3 according to an example of the disclosure.

In some examples, the sensor component 40 may include a sensor 41 (see FIG. 2A). The sensor 41 may be placed partially or completely subcutaneously in the host so that it may react with the analyte in the body fluid and generate an analyte signal. In this case, analyte information in the body fluid may be obtained by the sensor 41 reacting with the analyte in the body fluid. In some examples, the sensor 41 may include a head portion, a tail portion, and a connecting portion connecting the head portion and the tail portion. The head portion may include an electrically conductive region and is electrically connected to the electronic component 30 when the sensor component 40 is mounted to the electronic component 30, and the tail portion may include an implanted portion that is implantable subcutaneously in the host. Signals measured subcutaneously in the host with the tail portion arranged may be transmitted through the conductive region of the head portion to the electronic component 30.

In some examples, the sensor component 40 may include a connecting base 42 (see FIG. 2A). In some examples, the sensor 41 may be mounted to the electronic component 30 via a connection portion. In some examples, the sensor 41 may be electrically connected to the electronic component 30 via a connection portion.

In some examples, the sensor component 40 may include a sharp object 43 (see FIG. 2A). The sharp object 43 may be used to introduce the sensor 41 subcutaneously into the host. In some examples, the sensor 41 and the sharp object 43 may be arranged at the connecting base 42. In this case, by arranging both the sensor 41 and the sharp object 43 on the connecting base 42, it is possible to facilitate retaining both in a predetermined position, thereby facilitating alignment during subsequent pick-up of the sensor component 40. In some examples, the sharp object 43 may include a needle portion 431 and a carrier portion 432. The needle portion 431 may be used to accommodate the sensor 41, and the carrier portion 432 may be used to carry the needle portion 431. In some examples, the needle portion 431 may have a recessed structure 433 (see FIG. 2A) accommodating the sensor 41. In this case, the sharp object 43 facilitates guiding the sensor 41 into the subcutaneous tissue of the host during application of the patch component 3 to the host by the applicator 5. In some examples, the needle portion 431 may be fitted into the applicator 5 via the carrier portion 432.

In some examples, the connecting base 42 may have a sensor integrating hole. When the sensor 41 is coupled with the connecting base 42, the axis of the sensor 41 may pass through the sensor integrating hole. In this case, through the sensor integrating hole, the sharp object 43 is easily integrated with the sensor 41, thereby facilitating placement of the sensor 41 at least partially subcutaneously in the host via the sharp object 43.

In some examples, the electronic component 30 may include an outer casing 31 and a receiving portion 311 (see FIG. 2B). The receiving portion 311 may be used to receive the sensor component 40. In some examples, the receiving portion 311 may include an accommodating portion 312 and a through hole 314 (see FIG. 2B) that longitudinally extends through the outer casing 31. When the electronic component 30 is coupled to the sensor component 40, the connecting base 42 may be embedded in the accommodating portion 312, and the sharp object 43 may be at least partially positioned within the through hole 314. In this case, the stability of the component coupling between the electronic component 30 and the sensor component 40 may be improved, and during the pick-up process, it is easier for the applicator 5 to act on the electronic component to carry the sensor component 40 away from the cartridge device 1 together. Referring to FIGs. 2A and 2B, the electronic component 30 may move in direction D1 to achieve component coupling with the sensor component 40, forming the patch component 3 as shown in FIG. 2C, when the two components are coupled, the connecting base 42 may be embedded in the receiving portion 311.

In some examples, the connecting base 42 may include a structure for securely connecting to the electronic component 30. Here, a secure connection means that the connecting base 42 connected to the electronic component 30 will not separate from the electronic component 30 unless acted upon by a force greater than a predetermined magnitude. In some examples, the connecting base 42 may include an engagement portion 421 arranged on its outer periphery (see FIG. 2A), and the accommodating portion 312 may include a groove 313 that mates with the engagement portion 421 (see FIG. 2B); the engagement portion 421 and the groove 313 cooperate to achieve a secure coupling between the connecting base 42 and the electronic component 30. In some examples, the number of engagement portions 421 may be one or more, and the number of grooves 313 may correspond to the number of engagement portions 421. For example, the number of engagement portions 421 may be one, two, three, or four. In some examples, multiple engagement portions 421 may be distributed around the outer periphery of the connecting base 42. Thus, tight assembly between the connecting base 42 and the electronic component 30 may be facilitated.

In the disclosure, the cartridge device 1 may accommodate the sensor component 40. That is, the cartridge device 1 may be used to retain the sensor component 40.

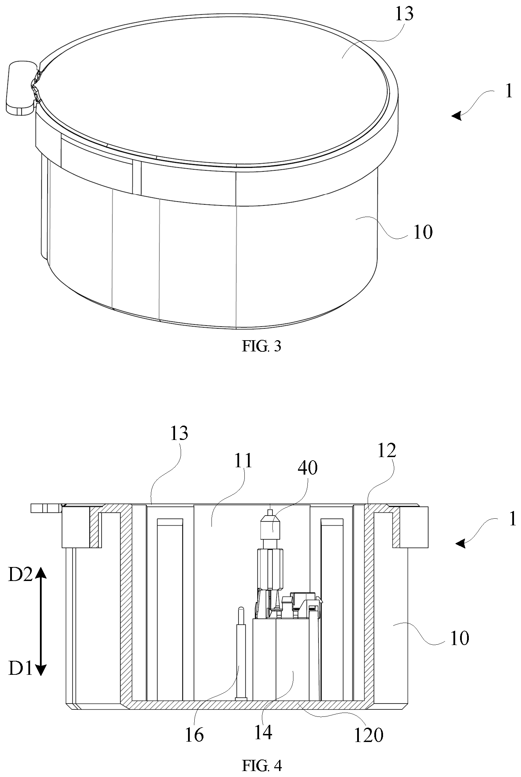

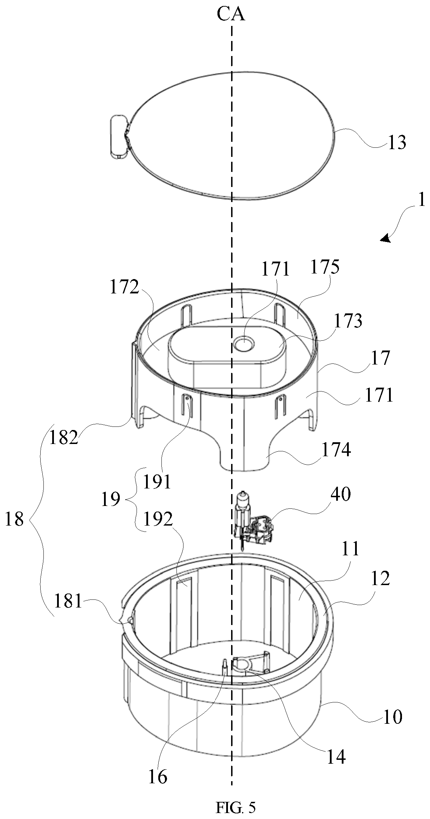

FIG. 3 is a schematic view showing the cartridge device 1 according to an example of the disclosure. FIG. 4 is a cross-sectional schematic view of the cartridge device 1 according to an example of the disclosure. In FIG. 4, certain members that may be confusing are omitted for clarity. FIG. 5 is an exploded schematic view of the cartridge device 1 according to an example of the disclosure. In FIG. 5, line CA schematically represents the central axis line of the cartridge device 1.

In some examples, the cartridge device 1 may include a housing 10 (see FIG. 3). The housing 10 may define an accommodating space 11. The sensor component 40 retained within the cartridge device 1 may be located in the accommodating space 11 (see FIG. 4).

In some examples, the cartridge device 1 may include an opening portion 12 (see FIG. 4). The accommodating space 11 may communicate with the exterior via the opening portion 12. In some examples, the cartridge device 1 may include a bottom portion 120 (see FIG. 4). The bottom portion 120 and the opening portion 12 may be located at opposite ends of the housing 10, respectively. For ease of understanding, the direction from the bottom portion 120 toward the opening portion 12 is subsequently considered upward, and the direction from the opening portion 12 toward the bottom portion 120 is subsequently considered downward. Referring to FIG. 4, direction D1 schematically indicates downward, and direction D2 schematically indicates upward. That is, the upper surface of a component arranged in the accommodating space 11 refers to the surface closer to the opening portion 12, and the lower surface refers to the surface closer to the bottom portion 120. In some examples, the pickup device may access the accommodating space 11 of the housing 10 via the opening portion 12 to pick up the sensor component 40, and remove the sensor component 40 from the cartridge device 1.

In some examples, the cartridge device 1 may include a sealing portion 13 (see FIG. 3). The sealing portion 13 may be used to seal the accommodating space 11. Specifically, the sealing portion 13 may be removably arranged at the opening portion 12 to seal the accommodating space 11. In this case, after sterilization, the accommodating space 11 is a sealed chamber, which may reduce contamination from the external environment, when use is required, removing the sealing portion 13 from the opening portion 12 places the interior of the housing 10 in an unsealed state, allowing operation on internal members. In some examples, the sealing portion 13 may be a flexible film, and the flexible film may be press-fitted onto the opening portion 12 to seal the accommodating space 11. Thus, it is possible to help maintain the sealed state of the cartridge device 1.

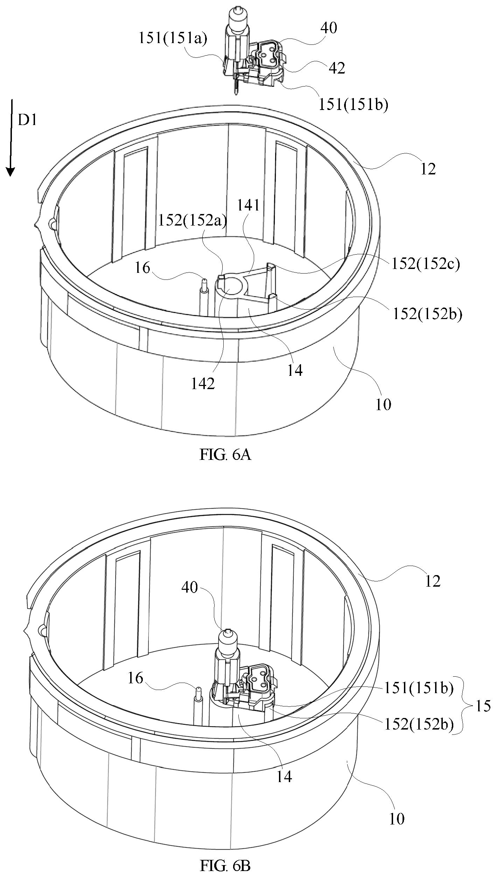

FIG. 6A is a schematic view showing the support mechanism 14 according to an example of the disclosure. FIG. 6B is a schematic view showing the sensor component 40 supported by the support mechanism 14 according to an example of the disclosure.

In some examples, the cartridge device 1 may include the support mechanism 14 (see FIG. 4). The support mechanism 14 may be used to support the sensor component 40, and the sensor component 40 may be retained within the cartridge device 1 via the support mechanism 14. In some examples, the support mechanism 14 may removably support the sensor component 40. That is, the sensor component 40 may be removed from the retention of the support mechanism 14 and move away from the support mechanism 14 under external force.

In some examples, the support mechanism 14 may be located in the accommodating space 11 of the housing 10 (see FIG. 6A). The sensor component 40 supported by the support mechanism 14 may also be located in the accommodating space 11. In some examples, the support mechanism 14 may be tightly integrated with the sensor component 40. In this case, the support mechanism 14 may restrict undesired movement of the sensor component 40, enabling the sensor component 40 to be stably arranged in the cartridge device 1, thereby facilitating alignment between the sensor component 40 and the pickup device during subsequent pickup operations.

In some examples, the cartridge device 1 may include a retaining portion 15 (see FIG. 6B). The retaining portion 15 may be used to tightly retain the sensor component 40. In some examples, the support mechanism 14 may retain the sensor component 40 via the retaining portion 15. In some examples, the retaining portion 15 may include male-female mechanical mating structures respectively arranged on the support mechanism 14 and the sensor component 40. In this case, the sensor component 40 may be tightly integrated with the support mechanism 14 by the male-female mechanical mating structure. In some examples, the male-female mechanical mating structure may include a concave structure 151 and a convex structure 152 that matches the concave structure 151. In other words, in some examples, the retaining portion 15 may include a concave structure 151 and a convex structure 152 that mates with the concave structure 151, and the concave structure 151 and the convex structure 152 may be arranged on the sensor component 40 and the support mechanism 14, respectively (see FIGs. 6A and 6B). In this case, by cooperating the convex structure 152 with the concave structure 151, the sensor component 40 may be integrated with the support mechanism 14, i.e., the sensor component 40 may be retained on the support mechanism 14; moreover, engagement facilitates tight integration between the sensor component 40 and the support mechanism 14; furthermore, the engagement structure is relatively simple, facilitating installation and manufacturing processes.

In some examples, the support mechanism 14 may include a support surface 141 (see FIG. 6A). The sensor component 40 retained by the support mechanism 14 may be in contact with the support surface 141 (see FIG. 6B). In some examples, optionally, the concave structure 151 may be arranged on the sensor component 40, and the convex structure 152 may be arranged on the support mechanism 14. Specifically, the retaining portion 15 may include a convex structure 152 protruding from the support surface 141, and a concave structure 151 arranged to be recessed inward from the outer surface of the sensor component 40 (refer to FIG. 6A). When the sensor component 40 is supported by the support mechanism 14, the concave structure 151 on the sensor component 40 may accommodate the convex structure 152 on the support mechanism 14. As a result, it is possible to facilitate stable support of the sensor component 40 by the support mechanism 14.

In some examples, the concave structure 151 may be recessed inwardly from the bottom of the connecting base 42, and the convex structure 152 may protrude upward from the support surface 141 (see FIG. 6A). Here, "protruding upward from the support surface 141" means protruding from the support surface 141 toward the direction close to the opening portion 12. In this case, the sensor component 40 may be moved downward onto the support mechanism 14. Referring to FIG. 6A, the sensor component 40 may be moved in direction D1 to integrate with the support mechanism 14 to form the configuration shown in FIG. 6B.

In some examples, the number of convex structures 152 and concave structures 151 may each be one or more, and the number of convex structures 152 and concave structures 151 may be equal. For example, the number of convex structures 152 and concave structures 151 may be one, two, three, four, five, or six. Referring to FIG. 6A, the number of convex structures 152 and concave structures 151 may each be three; the three convex structures 152 are convex structure 152a, convex structure 152b, and convex structure 152c, respectively. Due to the viewing angle, only two concave structures 151 - concave structure 151a and concave structure 151b - are visible in FIG. 6A. In some examples, a plurality of concave structures may be arranged around the outer periphery of the sensor component 40, with the plurality of convex structures 152 corresponding in position to the plurality of concave structures 151. In this case, the sensor 41 may be retained from multiple directions, thereby facilitating restriction of movement of the sensor component 40 on the support surface 141, further improving the stability of the sensor component 40 assembled to the support mechanism 14.

In some examples, the male-female mechanical mating structure may be a clearance fit and/or an interference fit. In some examples, the convex structure 152 and the concave structure 151 may be configured as a clearance fit and/or an interference fit. In some examples, in cases where the retaining portion 15 may include only one convex structure 152 and one concave structure 151, the convex structure 152 and the concave structure 151 may form an interference fit. In this case, it is possible to facilitate tight integration between the sensor component 40 and the support mechanism 14, thereby improving the stability of the sensor component 40 supported by the support mechanism 14. In some examples, in cases where the retaining portion 15 may include a plurality of convex structures 152 and a plurality of concave structures 151, at least one of the convex structures 152 may form an interference fit with its corresponding concave structure 151, while the others form clearance fits. In such a case, after tight integration between the sensor component 40 and the support mechanism 14, the force required to subsequently pick up the sensor component 40 and detach it from the support mechanism 14 may be reduced, thereby facilitating pickup. In some examples, in cases where the retaining portion 15 may include a plurality of convex structures 152 and a plurality of concave structures 151, all of the convex structures 152 and concave structures 151 may be interference fits.

In some examples, at least one of the plurality of convex structures 152 may be configured to be taller than the other convex structures 152, and the depth of the concave structure 151 corresponding to this convex structure 152 may also be greater than that of the other concave structures 151. As a result, the stability of the integration between the support mechanism 14 and the sensor component 40 may be further improved.

In some examples, the support mechanism 14 may include a hollow portion 142 (see FIG. 6A). The hollow portion 142 may be used to accommodate a portion of the sharp object 43 and the sensor 41 of the sensor component 40. Specifically, in the sensor component 40, a portion of the sharp object 43 and the sensor 41 may extend from the bottom of the connecting base 42. When the sensor component 40 is supported by the support mechanism 14, the bottom of the connecting base 42 may contact the support surface 141, and the portion of the sharp object 43 and the sensor 41 extending from the bottom of the connecting base 42 may be located within the hollow portion 142.

In some examples, the support mechanism 14 may be arranged on the bottom portion 120 and face the opening portion 12. In this case, the sensor component 40 retained by the support mechanism 14 may face the opening portion 12 to facilitate subsequent pickup of the sensor component 40 by the pickup device. In some examples, the support mechanism 14 may be integrally formed with the cartridge device 1. In this case, it is possible to reduce undesired movement of the support mechanism 14 under external force, improve the stability of the support mechanism 14, and thereby improve the stability of the sensor component 40 when retained by the support mechanism 14. In some examples, the convex structure 152 may be integrally formed with the support mechanism 14. In other words, the convex structure 152 may be regarded as part of the support mechanism 14.

In some examples, the cartridge device 1 may include an assist mechanism 16 (see FIG. 4). The assist mechanism 16 may provide a force to the pickup device away from the support mechanism 14 after the pickup device picks up the sensor component 40. That is, when the sensor component 40 moves away from the support mechanism 14 under the action of the pickup device coupled to the sensor component 40, the assist mechanism 16 may provide a force to the pickup device in a direction away from the support mechanism 14. In this case, the influence of the retention force exerted by the support mechanism 14 on the sensor component 40 upon the structural integrity of the pickup device itself may be reduced (i.e., the assisting force provided by the assist mechanism 16 may partially offset the retention force of the support mechanism 14), thereby facilitating the smooth separation of sensor component 40 tightly integrated with the support mechanism 14 from the support mechanism 14 under the action of the pickup device and improving the reliability of the pickup device in picking up the sensor component 40. In some examples, the assist mechanism 16 may be located within the accommodating space 11 of the housing 10 of the cartridge device 1.

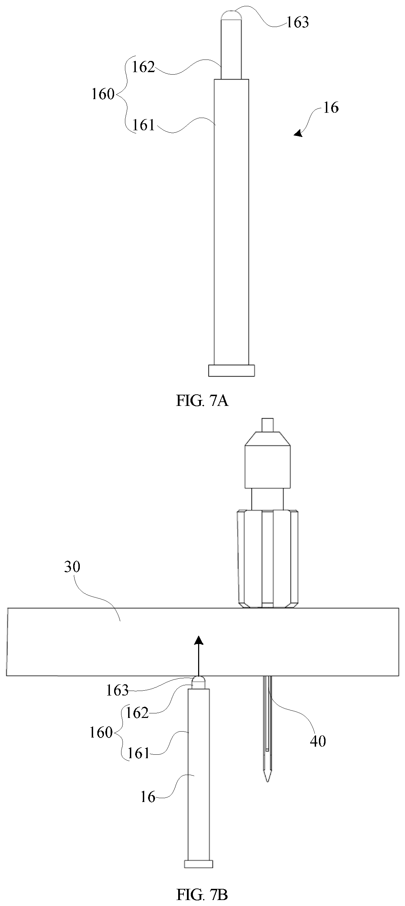

FIG. 7A is a schematic view showing the assist mechanism 16 according to an example of the disclosure in a natural state. FIG. 7B is a schematic view showing the assist mechanism 16 according to an example of the disclosure in a compressed state. In FIG. 7B, the assist mechanism 16 is compressed by the force from the pickup device. For clarity of illustration, other members of the cartridge device 1 and the applicator 5 are omitted, and only the electronic component 30 is shown for illustration, wherein the direction of the arrow schematically indicates the direction of the force provided by the assist mechanism 16 to the electronic component 30.

In some examples, the assist mechanism 16 may include a drive portion 160 (see FIG. 7A). During the process in which the pickup device carries the sensor component 40 away from the support mechanism 14 (hereinafter referred to as the "lift-off process"), the drive portion 160 abuts against a surface of the pickup device to provide a force to the pickup device away from the support mechanism 14.

In some examples, the drive portion 160 may include an elastic element. The elastic element may be in a compressed state when the pickup device picks up the sensor component 40 (see FIG. 7B), and an end portion of the elastic element may abut against a surface of the pickup device when the pickup device carries the sensor component 40 away from the support mechanism 14, thereby providing a force to the pickup device away from the support mechanism 14. In this case, when the pickup device carries the sensor component 40 away from the support mechanism 14, the release of stored elastic potential energy by the elastic element may provide a force to the pickup device away from the support mechanism 14. In some examples, the elastic element may be a spring or a leaf spring.

In some examples, when the sensor component 40 is retained in the standby state of the cartridge device 1, the elastic element may be in a free, uncompressed state; when the pickup device picks up the sensor component 40, the elastic element may be compressed by the pickup device and placed in a compressed state. Since prolonged compression may affect the performance of the elastic element, for example, causing undesirable non-elastic deformation (plastic deformation or material creep) that reduces the elastic potential energy storable in the elastic element, the disclosure configures the elastic element to remain in a free state during standby and to be compressed only during pickup, thereby reducing the likelihood of reduced storable elastic potential energy due to prolonged compression, and thus enabling stable application of a force away from the support mechanism 14 to the pickup device during pickup.

In some examples, the drive portion 160 may include an actuating member 162 (see FIG. 7A). The actuating member 162 may be connected to the elastic element, and force may be transmitted through contact between the actuating member 162 and the pickup device. In some examples, the actuating member 162 may be generally columnar in shape.

In some examples, the drive portion 160 may be a spring probe structure (see FIG. 7A). Specifically, the drive portion 160 may include an accommodating casing 161 having an storage space, and an actuating member 162 connected to the elastic element, wherein the elastic element is arranged within the accommodating casing 161 with its elastic direction aligned with the longitudinal direction of the accommodating casing 161, a first end of the actuating member 162 connected to the elastic element is located within the storage space, while the second end 163 extends out of the accommodating casing 161, and force transmission may be achieved via contact between the second end 163 of the actuating member 162 and the pickup device. In this case, the spring probe structure enables effective force transmission and occupies a small overall space, facilitating miniaturization of the cartridge device 1.

In some examples, the outer contour of the portion of the drive portion 160 that contacts the pickup device may be a curved surface (see FIG. 7A). In this case, damage that may be caused to the pickup device by the force applied from the drive portion 160 may be reduced, while maintaining effective force transmission. For example, in an example where the drive portion 160 is a spring probe structure, the outer contour of the second end 163 of the actuating member 162 may be a curved surface.

In some examples, the assist mechanism 16 may be adjacent to the support mechanism 14 (see FIG. 4). In this case, interaction between the pickup device and the assist mechanism 16 may be facilitated when the pickup device picks up the sensor component 40 located on the support mechanism 14. In some examples, the assist mechanism 16 may be arranged at the bottom portion 120. In this case, it may be convenient for the assist mechanism 16 to provide a force to the pickup device directed away from the support mechanism 14.

In some examples, the upper surface of the drive portion 160 of the assist mechanism 16 in its uncompressed state may be higher than the support surface 141 of the support mechanism 14. Here, the upper surface of the drive portion 160 refers to the surface of the drive portion 160 closest to the opening portion 12. As a result, the drive portion 160 may apply an assisting force to the pickup device throughout the entire process of the sensor component 40 disengaging from the support mechanism 14. In some examples, where the retaining portion 15 may include a plurality of concave structures 151 and convex structures 152, and the convex structures 152 are arranged on the support mechanism 14, the upper surface of the drive portion 160 of the assist mechanism 16 in its uncompressed state may be at least higher than the upper surface of one convex structure 152. In this case, at least during the disengagement of the sensor component 40 from the convex structure 152, the assist mechanism 16 may provide an upward assisting force to the pickup device to ensure stable disengagement of the sensor component 40 from the support mechanism 14.

In some examples, both the assist mechanism 16 and the support mechanism 14 may face the opening portion 12. That is, the sensor component 40 may be assembled to the support mechanism 14 by passing through the opening portion 12 into the housing 10 of the cartridge device 1, and the distance between the sensor component 40 supported by the support mechanism 14 and the opening portion 12 is smaller than the distance between the support mechanism 14 and the opening portion 12. In this case, the pickup device passes through the opening portion 12 of the cartridge device 1 into the accommodating space 11 of the housing 10 to retrieve the sensor component 40, and the assist mechanism 16 applies a force to the pickup device in a direction toward the opening portion 12 upon exiting, which helps the pickup device carry the sensor component 40 away from the support mechanism 14.

As previously mentioned, in some examples, the pickup device may include an applicator 5 that is removably retained with the electronic component 30. The sensor component 40 may be removed by coupling the applicator 5 to the cartridge device 1, thereby coupling the electronic component 30 and the sensor component 40 to form the patch component 3. At this point, the cartridge device 1 may be part of an analyte monitoring system 100.

In some examples, the applicator 5 may be coupled to the cartridge device 1 to pick up the sensor component 40 such that the sensor component 40 is coupled to the electronic component 30 to form the patch component 3. In some examples, the assist mechanism 16 may provide a force to the electronic component 30 directed away from the support mechanism 14. In this case, after the applicator 5 couples the electronic component 30 and the sensor component 40 to form the patch component 3, the force applied by the applicator 5 to the electronic component 30 in a direction away from the support mechanism 14 is transmitted to the sensor component 40 through the electronic component 30, conversely, when the sensor component 40 disengages from the support mechanism 14, the retention force exerted by the support mechanism 14 on the sensor component 40 is transmitted to the electronic component 30 via the sensor component 40, at this time, the electronic component 30, which is removably retained on the applicator 5, is simultaneously subjected to a force directed away from the support mechanism 14 and a resistance force derived from the retention force of the support mechanism 14, the force provided by the assist mechanism 16 to the electronic component 30 may offset part of this resistance, thereby reducing the likelihood of undesirable displacement or even detachment of the electronic component 30 from the applicator 5 due to this resistance, thus improving the reliability of the applicator 5 in picking up the sensor component 40 from the cartridge device 1 via the removably retained electronic component 30.

In some examples, during the removal of the patch component 3 from the support mechanism 14, the drive portion 160 contacts the surface of the electronic component 30 facing away from the applicator 5, providing a force to the electronic component 30 directed away from the support mechanism 14. Here, the surface of the electronic component 30 facing away from the applicator 5 refers to the surface of the electronic component 30 facing the opening of the applicator 5 (this surface is hereinafter referred to as "the bottom surface of the electronic component 30"). In such a case, when the drive portion 160 provides a force to the electronic component 30 away from the support mechanism 14 to partially counteract the resistance from the support mechanism 14, this force may also help press the electronic component 30 toward the applicator 5, further enhancing the stability with which the electronic component 30 is retained in the applicator 5.

In some examples, the elastic element of the drive portion 160 may be in a compressed state when the applicator 5 picks up the sensor component 40; During the removal of the patch component 3 from the support mechanism 14, the end portion of the drive portion 160 may contact the bottom surface of the electronic component 30 to provide a force to the electronic component 30 directed away from the support mechanism 14. In this case, after pickup is completed, the elastic element in the compressed state stores elastic potential energy, and during the separation of the patch component 3 from the support mechanism 14, the contact between the drive portion 160 including the elastic element and the surface of the electronic component 30 may utilize the elastic potential energy to provide a force to the electronic component 30 directed away from the support mechanism 14.

In some examples, the bottom surface of the electronic component 30 may be a surface having an adhesive (hereinafter simply referred to as an adhesive surface), as previously described. In some examples, the contact area of the drive portion 160 with the adhesive surface accounts for no more than 10% of the total area of the adhesive surface. In this case, on the basis of effectively achieving force transmission between the assist mechanism 16 and the electronic component 30, the pulling force on the electronic component 30 due to adhesion when the drive portion 160 detaches from the adhesive surface may be reduced, thereby facilitating the stable retention of the electronic component 30 in the applicator 5.

In some examples, optionally, the contact area of the drive portion 160 with the adhesive surface accounts for no more than 5% of the total area of the adhesive surface. For example, the contact area of the drive portion 160 with the adhesive surface may be 1%, 2%, 3%, 4%, or 5% of the total area of the adhesive surface. As a result, the adhesion force generated when the drive portion 160 contacts the adhesive surface may be further reduced. In the example where the drive portion 160 is a spring probe structure as previously described, since the actuating member 162 has a small volume, the area of the contact portion between the drive portion 160 and the adhesive surface may be reduced, thereby reducing adhesion between the drive portion 160 and the adhesive surface.

In some examples, the bottom surface of the electronic component 30 may include a first region having adhesiveness and a second region. The adhesiveness of the second region may be weaker than that of the first region, and the drive portion 160 may contact the second region. In this case, arranging the bottom surface of the electronic component 30 with a first region having adhesiveness enables the electronic component 30 to detach from the applicator 5 and retain on the host's body surface after being applied to the host's body surface through adhesion; additionally, by arranging a second region with adhesiveness weaker than the first region and configuring the drive portion 160 to contact the second region, the pulling force on the electronic component 30 caused by adhesion during separation of the drive portion 160 from the second region may be reduced on the basis of effective force transmission, thereby facilitating stable retention of the electronic component 30 in the applicator 5.

In some examples, the second region may have no adhesiveness. In this case, separation of the drive portion 160 from the second region does not generate a pulling force due to adhesion. In some examples, the first region may be located at the outer periphery of the second region. In this case, the drive portion 160 acting near the center of the bottom surface of the electronic component 30 enables more convenient force transmission, thereby facilitating smooth and stable retention of the electronic component 30 in the applicator 5 during the separation process. It should be noted that a second region without adhesiveness may be considered to have zero adhesiveness and still falls within the scope of "a second region less adhesive than the first region" as described in this disclosure.

In some examples, the outer contour of the portion of the drive portion 160 in contact with the electronic component 30 may be a curved surface. In this case, on the basis of effectively achieving force transmission, the contact area between the drive portion 160 and the electronic component 30 may be reduced, thereby reducing any pulling force on the electronic component 30 due to adhesion during separation of the drive portion 160 and the electronic component 30, allowing the stable retention of the electronic component 30 in the applicator 5; additionally, compared to a sharp surface, forming the contact surface as a curved surface may also reduce potential damage to the electronic component 30 when the drive portion 160 applies a force to the electronic component 30.

In some examples, the applicator 5 retaining the electronic component 30 may enter the accommodating space 11 of the cartridge device 1, move downward to a pickup position, so that the electronic component 30 couples with the sensor component 40, and at this time, the bottom surface of the electronic component 30 contacts the assist mechanism 16 and compresses it; after pickup is completed, the applicator 5 moves upward together with the patch component 3, while the assist mechanism 16 exerts an upward assisting force on the electronic component 30, causing the sensor component 40 to disengage from the support mechanism 14; once the applicator 5 completely exits the accommodating space 11 of the cartridge device 1, the pickup operation is completed.

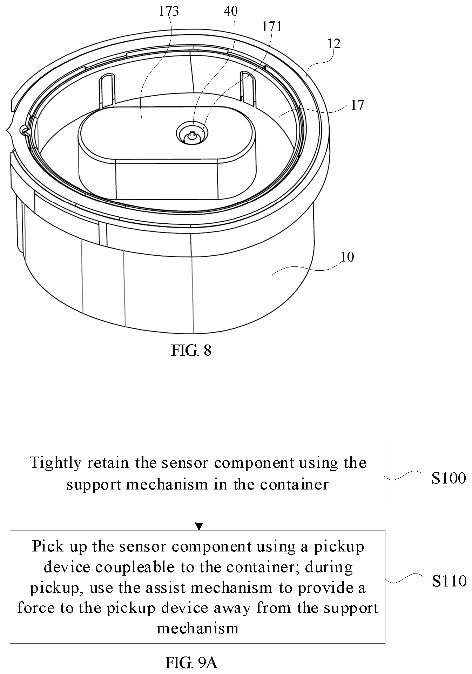

FIG. 8 is a schematic view showing the limiting cover 17 positioned in the housing 10 according to an example of the disclosure.

In some examples, the cartridge device 1 may include a limiting cover 17 (see FIG. 5). The limiting cover 17 may be used to limit the position of the sensor component 40. In some examples, the limiting cover 17 may be located in the accommodating space 11 of the housing 10. Sloshing of the cartridge device 1 may occur during transport, in which case unwanted displacement and/or rotation of the sensor component 40 may be further limited by the limiting cover 17 to maintain the sensor component 40 in a predetermined position to facilitate alignment during pick-up by a subsequent pickup device.

In some examples, the limiting cover 17 may be placed over the sensor component 40 (see FIG. 8). When the limiting cover 17 is moved away from the accommodating space 11, the sensor component 40 may be brought into a pick-up state. In the prior art, there is a solution of protecting the sensor by using a stopper platform that may move up and down in the cartridge device, further fixing the stopper platform around the sensor component when it is above the sensor component, and pressing the stopper platform down to unlock the sensor component through cooperation between the applicator and the stopper platform to expose it for pickup by the applicator; however, such an unlocking and cooperating structure is relatively complicated and requires designing various structures such as guide slots and latches. Therefore, unlike such a structure, in the disclosure, configuring the limiting cover 17 to move away from the accommodating space 11 so that the sensor component 40 may be in a pickable state simplifies the complicated cooperating structure, and the housing 10 of the cartridge device 1 does not need to be designed with a large depth to allow movement of the stopper platform, facilitating miniaturization of the cartridge device 1.

In some examples, the limiting cover 17 may include a limiting structure 171 (see FIG. 5). The limiting structure 171 may be arranged on the outer periphery of at least part of members of the sensor component 40 in a manner that does not contact the sensor component 40. In this case, compared to direct contact with the sensor component 40, arranging the limiting structure 171 around the outer periphery of the sensor component 40 without contacting it reduces the possibility of undesirable displacement of the sensor component 40 caused by friction between the two due to movement of the limiting structure 171 (e.g., when removing the limiting cover 17).

In some examples, in cases where the cartridge device 1 may include both the support mechanism 14 and the limiting cover 17, the sensor component 40 may be tightly integrated with the support mechanism 14, and the limiting cover 17 may be arranged around the outer periphery of the sensor component 40 in a manner that does not contact the sensor component 40. As a result, the sensor component 40 may be stably retained. Furthermore, after removal of the limiting cover 17, the sensor component 40 may still be tightly retained within the cartridge device 1 by the support mechanism 14, thereby facilitating a subsequent pickup operation.

In some examples, the limiting cover 17 may include a cover body 172, and the limiting structure 171 may be a channel formed in the cover body 172 (see FIG. 5). When the limiting cover 17 is placed over the sensor component 40, the sensor component 40 may be at least partially positioned within the channel (see FIG. 8), and the inner wall of the cover body 172 may surround the outer periphery of the sensor component 40. In this case, the likelihood of undesirable displacement of the sensor component 40 due to overall shaking of the cartridge device 1 may be reduced.

In some examples, the limiting cover 17 may include a grip portion 173 (see FIG. 5). The grip portion 173 may be a projection formed on the cover body 172 and extending toward the opening portion 12. In this case, applying force to the grip portion 173 facilitates removal of the limiting cover 17 from the accommodating space 11. In some examples, the limiting structure 171 may be a channel formed in the grip portion 173. In this case, manufacturing of the limiting cover 17 may be facilitated without requiring additional design of protrusion structures for forming the limiting channel.

In some examples, the cover body 172 may include an upper leg 175 (see FIG. 5) that extends in a direction toward the opening portion 12. In some examples, the cover body 172 may include a lower leg 174 (see FIG. 5) that extends toward the bottom portion 120. In some examples, the lower leg 174 may abut the bottom portion 120 of the cartridge device 1 or a member arranged on the bottom portion 120. Thus, the limiting cover 17 may be stably retained in the housing 10. In some examples, the upper leg 175 and/or the lower leg 174 may extend in a predetermined direction from the outer periphery of the cover body 172, and the upper leg 175 and/or the lower leg 174 may be in contact with the inner wall of the housing 10. In this case, it is possible to make the limiting cover 17 have a large contact area with the housing 10, thereby helping to suppress an undesired movement of the limiting cover 17 within the housing 10.

In some examples, the cartridge device 1 may include a first guide structure 18 (see FIG. 5). The first guide structure 18 may guide the limiting cover 17 to move in a desired direction within the housing 10. In this case, on the one hand, assembly of the two may be facilitated, and on the other hand, removal of the limiting cover 17 without touching the sensor component 40 may be facilitated by guiding the limiting cover 17 when it is removed. In some examples, the first guide structure 18 may be a male-female mechanical mating structure arranged on the housing 10 and the limiting cover 17, respectively. For example, the first guide structure 18 may include a first guide slot 181 arranged on the housing 10, and a first rib 182 arranged on the limiting cover 17 (see FIG. 5). In this case, the direction of movement of the limiting cover 17 within the housing 10 may be guided by the cooperation of the first rib 182 and the first guide slot 181. In some examples, the first guide structure 18 may include a first guide slot 181 arranged on the housing 10, and a protrusion arranged on the limiting cover 17. Thus, the movement direction of the limiting cover 17 within the housing 10 may be guided by the cooperation of the protrusion and the first guide slot 181.

In some examples, the cartridge device 1 may include a retention mechanism 19 (see FIG. 5). The retention mechanism 19 may be used to retain the limiting cover 17. Specifically, the retention mechanism 19 may be configured to provide a predetermined amount of retention force to the limiting cover 17, the retention force being greater than the gravitational force of the limiting cover 17. The cartridge device 1 as a whole may be shaken or even turned upside down during transport or when the user opens the cartridge device 1, in which case the use of the retention mechanism 19 to retain the limiting cover 17 may reduce the occurrence of undesired movements of the limiting cover 17 in the housing 10 or even falling out of the housing 10.

In some examples, the retention mechanism 19 may be a male-female mechanical mating structure arranged on the limiting cover 17 and the housing 10, respectively. For example, the retention mechanism 19 may include a biasing arm 191 that may be biased away from and/or toward the central axis line CA of the cartridge device 1, and a recess 192 that cooperates with the biasing arm 191 (see FIG. 5). The biasing arm 191 may be at least partially located in the recess 192 when the limiting cover 17 is located inside the housing 10; when it is desired to remove the limiting cover 17 from the housing 10, a force applied to the limiting cover 17 away from the housing 10 may cause the biasing arm 191 to move away from the recess 192. In some examples, when the biasing arm 191 is arranged at the limiting cover 17 and the recess 192 is arranged at the housing 10, the biasing arm 191 may have a tendency (i.e., a tendency to expand outward) away from the central axis line CA of the cartridge device 1. Thus, after the limiting cover 17 enters the housing 10, the biasing arm 191 may automatically expand outward into the recess 192 to retain the limiting cover 17. Conversely, when the biasing arm 191 is arranged in the housing 10 and the recess 192 is arranged in the limiting cover 17, the biasing arm 191 may tend to move close to the central axis line CA of the cartridge device 1 (i.e., a tendency to fold inward). In some examples, the surface of the biasing arm 191 in contact with the recess 192 may be a curved surface. Thereby, the movement of the biasing arm 191 into and out of the recess 192 may be facilitated.

In some examples, the cartridge device 1 may include a platform. The platform may have a storage space. In some examples, a desiccant may be stored in the storage space of the platform. Thereby, it is possible to contribute to a dry environment within the cartridge device 1, thereby facilitating the preservation of the sensor component 40. In some examples, the platform may be arranged on the bottom portion 120, and the support mechanism 14 and the assist mechanism 16 may be arranged on the bottom of the housing 10 and exposed through the platform. In this case, the stability of the support mechanism 14 and the assist mechanism 16 may be further improved by the platform. In some examples, the lower leg 174 of the limiting cover 17 may be in contact with the platform such that the entire limiting cover 17 is mounted over the platform.

In summary, in the first aspect of the disclosure, a cartridge device 1 is provided that may stably retain the sensor component 40 and improve the reliability of the pickup device in picking up the sensor component 40.

A second aspect of the disclosure relates to a pickup method for the sensor component 40 (referred to as a pickup method for short). In the disclosure, the pickup method may also be referred to as an assembly method, an assembling method, or the like.

FIG. 9A is a first flowchart showing a pickup method according to an example of the disclosure.

In the present embodiment, the pickup method may include: tightly retaining the sensor component using the support mechanism in the container (step S100); picking up the sensor component using a pickup device coupleable to the container, during the pickup process, using the assist mechanism to provide a force to the pickup device away from the support mechanism (step S110) (see FIG. 9A). In this case, by tightly integrating the support mechanism in the container with the sensor component to removably retain the sensor component, the sensor component may be stably arranged in the container, and alignment between the sensor component and the pickup device may be facilitated when subsequently using the pickup device to pick up the sensor component; by providing a force to the pickup device away from the support mechanism upon pickup of the sensor component by the pickup device from the assist mechanism, the influence of the retention force exerted by the support mechanism on the sensor component upon the structural integrity of the pickup device itself may be reduced (i.e., the assisting force provided by the assist mechanism may partially offset the retention force of the support mechanism), thereby facilitating the smooth separation of the sensor component tightly integrated with the support mechanism from the support mechanism under the action of the pickup device and improving the reliability of the pickup device in picking up the sensor component. As a result, a high-reliability pickup method for the sensor component may be provided.

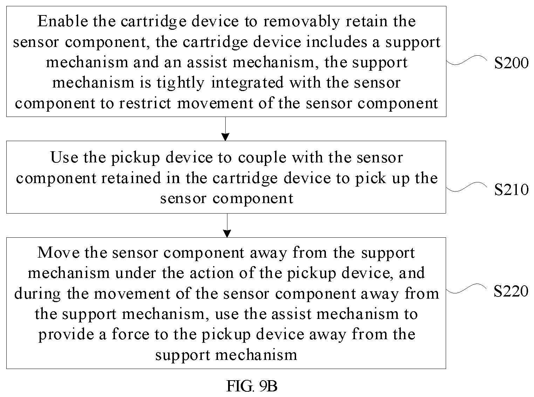

FIG. 9B is a second flowchart showing a pickup method according to an example of the disclosure.

In some examples, the container described in the pickup method of the disclosure may refer to the cartridge device 1 of the first aspect of the disclosure, and the specific components and structures (e.g., the support mechanism 14 and the assist mechanism 16) are the same as those of the cartridge device 1 in the first aspect of the disclosure and will not be described again herein. That is, the pickup method of the disclosure may include: enabling the cartridge device 1 to removably retain the sensor component 40, the cartridge device 1 may include a support mechanism 14 and an assist mechanism 16, and the support mechanism 14 is tightly integrated with the sensor component 40 to restrict movement of the sensor component 40 (Step S200); using the pickup device to couple with the sensor component 40 retained in the cartridge device 1 to pick up the sensor component 40 (step S210); moving the sensor component 40 away from the support mechanism 14 under the action of the pickup device, and during the movement of the sensor component 40 away from the support mechanism 14, using the assist mechanism 16 to provide a force to the pickup device away from the support mechanism 14 (step S220) (see FIG. 9B). Thus, a high-reliability pickup method of the sensor component 40 may be provided.

A third aspect of the disclosure is directed to an analyte monitoring system 100. The analyte monitoring system 100 according to the third aspect of the disclosure is the same as the analyte monitoring system 100 described in connection with the cartridge device 1 in the first aspect of the disclosure; previously described content will not be repeated here.

As previously mentioned, in some examples, the analyte monitoring system 100 may include a patch component 3, an applicator 5, and a cartridge device 1. Here, the cartridge device 1 and the patch component 3 are identical to those of the cartridge device 1 and the patch component 3 described in the first aspect of the disclosure, and specific structural components may be found in the foregoing description and will not be repeated herein.

That is, in some examples, the analyte monitoring system 100 may include a sensor component 40, a cartridge device 1 removably retaining the sensor component 40, an electronic component 30, and an applicator 5 removably retaining the electronic component 30. The cartridge device 1 may include a support mechanism 14 removably supporting the sensor component 40, and an assist mechanism 16, wherein the support mechanism 14 is tightly integrated with the sensor component 40 to restrict movement of the sensor component 40; the applicator 5 cooperates with the cartridge device 1 to couple the electronic component 30 with the sensor component 40 to form the patch component 3; when the sensor component 40 moves away from the support mechanism 14 under the action of the electronic component 30 and the applicator 5, the assist mechanism 16 provides a force to the electronic component 30 away from the support mechanism 14. In this case, the cartridge device 1 may include the support mechanism 14 and the assist mechanism 16, the tight integration between the support mechanism 14 and the sensor component 40 enables the sensor component 40 to be stably arranged in the cartridge device 1, and facilitates alignment between the sensor component 40 and the applicator 5 during subsequent pickup of the sensor component 40 by the applicator 5; by means of the assist mechanism 16 providing a force away from the support mechanism 14 to the electronic component 30 when the applicator 5 leaves the support mechanism 14 after picking up of the sensor component 40, the influence of the retention force exerted by the support mechanism 14 on the sensor component 40 upon the structural integrity of the pickup device itself may be reduced (i.e., the assisting force provided by the assist mechanism 16 may partially offset the retention force of the support mechanism 14), reducing the likelihood of undesirable displacement or even detachment of the electronic component 30 from the applicator 5 due to such resistance, thereby facilitating the smooth separation of the sensor component 40 tightly integrated with the support mechanism 14 from the support mechanism 14 under the action of the applicator 5 and improving the reliability of the applicator 5 in picking up the sensor component 40. Thus, an analyte monitoring system 100 with high reliability in the pickup process of the sensor component 40 may be provided.

In some examples, as previously described, the sensor component 40 may include a connecting base 42, and a sensor 41, and a sharp object 43 arranged on the connecting base 42. In this case, the cartridge device 1 may accommodate the sensor 41 and the sharp object 43 to be inserted into the host, allowing simultaneous sterilization; and by mounting both the sensor 41 and the sharp object 43 on the connecting base 42, they may be retained at a predetermined position, thereby facilitating alignment during subsequent pickup operations.

In some examples, as previously described, the electronic component 30 may include an outer casing 31 and a receiving portion 311 for receiving the sensor component 40, the receiving portion 311 including an accommodating portion 312 and a through hole 314 extending longitudinally through the outer casing 31; when the electronic component 30 is coupled to the sensor component 40, the connecting base 42 is embedded in the accommodating portion 312 and the sharp object 43 is at least partially located within the through hole 314. In this case, the stability of the coupling between the electronic component 30 and the sensor component 40 may be improved, and during the pick-up process, the applicator 5 may facilitate acting on the electronic component to carry the sensor component 40 away from the support mechanism 14 together.

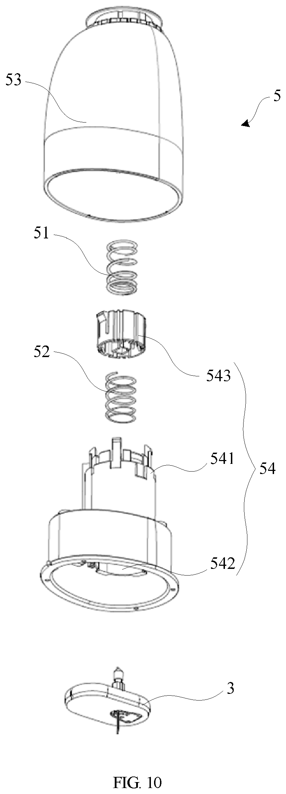

In some examples, the electronic component 30 may include an electronic device. The electronic device may be arranged within the outer casing 31. In some examples, the electronic device may include a printed circuit board. The printed circuit board may be used to receive physiological information of the host measured by the sensor 41, process the information, and transmit it to the smart device. FIG. 10 is an exploded schematic view showing an applicator 5 according to an example of the disclosure.

In some examples, the applicator 5 may include a proximal end that is close to the host and a distal end that is away from the host during operation. In some examples, the applicator 5 may include a first drive mechanism 51 (see FIG. 10). The first drive mechanism 51 may be configured to apply a force toward the proximal end to the patch component 3, driving the patch component 3 toward the proximal end via the first drive mechanism 51 so that the sensor 41 is placed at least partially subcutaneously in the host through the sharp object 43.

In some examples, the applicator 5 may include a second drive mechanism 52 (see FIG. 10). After the sharp object 43 reaches the predetermined position, it may be driven toward the distal end by the second drive mechanism 52, causing the sharp object 43 to withdraw from the host. In the disclosure, the sensor component 40 and the electronic component 30, which are applied to the surface of the host's body and may not include the sharp object 43, may be referred to as a sensor control device.

In some examples, the applicator 5 may include a first drive mechanism 51 and a second drive mechanism 52. Thus, by using two drive mechanisms, the patch component 3 may be applied to the host, and the sharp object 43 may be withdrawn from the host.

In some examples, the applicator 5 may include an applicator housing 53 (see FIG. 10). The applicator housing 53 may have a storage space. The electronic component 30 retained in the applicator 5 may be located within the storage space of the applicator housing 53.

In some examples, the applicator housing 53 may enter the accommodating space 11 of the cartridge device 1 together with the electronic component 30 to pick up the sensor component 40. In some examples, the applicator housing 53 may include a flange. The flange may cooperate with the first guide slot 181 on the housing 10 of the cartridge device 1, enabling the applicator housing 53 to move along a desired direction within the housing 10 of the cartridge device 1. In this case, the coupling of the two components may be facilitated, and since the applicator housing 53 and the limiting cover 17 share the same guide slot, the redundant design may be reduced.

In some examples, the applicator 5 may include an auxiliary mechanism 54 (see FIG. 10) configured to be movable relative to the applicator housing 53. The applicator housing 53 may have a proximal end that is close to the host during operation and a distal end that is away from the host. The auxiliary mechanism 54 may be removably retained to the applicator housing 53 and may store the patch component 3. In some examples, after being released, the auxiliary mechanism 54 may move along a predetermined path and apply the patch component 3 to a predetermined position.

In some examples, the auxiliary mechanism 54 may include a moving body 541, a first storage portion 542, and a second storage portion 543 (see FIG. 10). The moving body 541 may be removably retained within the applicator housing 53, the first storage portion 542 may removably retain the electronic component 30, and the second storage portion 543 may retain the sharp object 43. After the moving body 541 is released, it moves toward the proximal end of the applicator housing 53, and the first storage portion 542 and the second storage portion 543 also move toward the proximal end of the applicator housing 53, with the sharp object 43 carrying the sensor 41 piercing subcutaneously into the host, thereby placing the sensor 41 at least partially under the skin of the host. In some examples, the second storage portion 543 may removably retain the sharp object 43.

In some examples, the first drive mechanism 51 may be configured to apply a force toward the proximal end on the moving body 541. When the moving body 541 is released, the moving body 541 may be driven toward the proximal end by the first drive mechanism 51 to push the patch component 3 accommodated in the first storage portion 542 and the second storage portion 543 toward the host. In other examples, the applicator 5 may not include the first drive mechanism 51. In this case, when the moving body 541 is released, the moving body 541 may also be manually driven to move toward the proximal end.

In some examples, the second storage portion 543 may be releasably arranged on the moving body 541, and the second storage portion 543 may be configured to move relative to the first storage portion 542 when released. For example, when the second storage portion 543 is released, it may move relative to the first storage portion 542 in a direction away from the host. In this case, after the sharp object 43 retained by the second storage portion 543 penetrates subcutaneously into the host together with the sensor 41 to a predetermined position, the sharp object 43 may be moved in a direction away from the host under the action of the second storage portion 543 to retract the sharp object 43 from the subcutaneous tissue of the host and separate it from the electronic component 30. In some examples, the second storage portion 543 may retain the sharp object 43 by clamping the carrier portion 432 of the sharp object 43 that has an annular groove.

In some examples, when the second storage portion 543 is released, the second storage portion 543 may be driven toward the distal end by the second drive mechanism 52 to move the sharp object 43 away from the host. In other examples, the applicator 5 may not include the second drive mechanism 52. In this case, the second storage portion 543 may be manually driven away from the host. In some examples, the second storage portion 543 may also be integrally connected with the moving body 541, and the sharp object 43 may be moved away from the host by manually moving the moving body 541 in a direction away from the host.

In the disclosure, various functional modules within the applicator 5 may be designed according to conventional practices in the art, as long as they enable the function of pushing the patch component 3 onto the host; such details are not further elaborated herein. In view of the foregoing, a third aspect of the disclosure may provide an analyte monitoring system 100 with high reliability in the pickup process of the sensor component 40.

In view of the above, according to the disclosure, it is possible to provide a cartridge device 1, a pickup method for the sensor component 40, and an analyte monitoring system 100, all featuring high reliability in the pickup process of the sensor component 40.

Although the disclosure has been specifically described with reference to the accompanying drawings and examples, it should be understood that the above description does not limit the disclosure in any form. Those skilled in the art may make modifications and variations to the disclosure without departing from its essential spirit and scope, and such modifications and variations fall within the scope of the disclosure.

## Claims

1. A cartridge device, for retaining a sensor component, wherein,
the cartridge device comprises a housing having an accommodating space, a support mechanism removably supporting the sensor component, and an assist mechanism, the support mechanism, the assist mechanism, and the sensor component supported by the support mechanism are located in the accommodating space,
the support mechanism is tightly integrated with the sensor component to restrict movement of the sensor component, and the assist mechanism provides a force to the pickup device away from the support mechanism when the sensor component moves away from the support mechanism under the action of the pickup device coupled to the sensor component.

2. The cartridge device according to claim 1, wherein,
the pickup device comprises an applicator removably retaining an electronic component, the applicator is coupled to the cartridge device to pick up the sensor component such that the sensor component and the electronic component are coupled to form a patch component, the assist mechanism provides a force to the electronic component away from the support mechanism.

3. The cartridge device according to claim 2, wherein,
the assist mechanism comprises a drive portion, during removal of the patch component from the support mechanism, the drive portion abuts a surface of the electronic component facing away from the applicator to provide a force to the electronic component away from the support mechanism.

4. The cartridge device according to claim 3, wherein,
the drive portion comprises an elastic element, the elastic element is in a compressed state when the applicator picks up the sensor component, during removal of the patch component from the support mechanism, an end portion of the drive portion contacts a surface of the electronic component facing away from the applicator to provide a force to the electronic component away from the support mechanism.

5. The cartridge device according to claim 3, wherein,
the surface of the electronic component facing away from the applicator is an adhesive surface, and a contact area of the drive portion with the adhesive surface accounts for not more than 10% of the total area of the adhesive surface.

6. The cartridge device according to claim 3, wherein,
a surface of the electronic component facing away from the applicator comprises a first region having adhesiveness, and a second region having a weaker adhesiveness than the first region, the first region is located at an outer periphery of the second region, the drive portion is in contact with the second region.

7. The cartridge device according to claim 5 or 6, wherein,
an outer contour of a portion of the drive portion in contact with the electronic component is a curved surface.

8. The cartridge device according to claim 1,
further comprising a retaining portion for tightly retaining the sensor component, the retaining portion being a male-female mechanical mating structure respectively arranged on the support mechanism and the sensor component, the male-female mechanical mating structure being a clearance fit and/or an interference fit.

9. The cartridge device according to claim 1,
further comprising a limiting cover located in the accommodating space, the limit cover being arranged over the sensor component and having a limiting structure arranged around an outer periphery of at least part of members of the sensor component in a manner that does not contact the sensor component, and after the limiting cover leaving the accommodating space, the sensor component being in a pickable state.

10. The cartridge device according to claim 9,
further comprising a retention mechanism for retaining the limiting cover, the retention mechanism being configured to provide a predetermined retention force to the limiting cover, the retention force being greater than a gravitational force of the limiting cover.

11. The cartridge device according to claim 1,
further comprising an opening portion and a sealing portion, the accommodating space of the housing communicating with exterior via the opening portion, the sealing portion being detachably arranged at the opening portion to seal the accommodating space.

12. A pickup method of a sensor component,
comprising:
tightly and removably retaining the sensor component using a support mechanism in a container;
picking up the sensor component using a pickup device coupleable to the container, and during pickup, applying a force to the pickup device away from the support mechanism using an assist mechanism.

13. An analyte monitoring system,
comprising a sensor component, a cartridge device removably retaining the sensor component, an electronic component, and an applicator removably retaining the electronic component,
the cartridge device comprising a support mechanism removably supporting the sensor component, and an assist mechanism, the support mechanism being tightly integrated with the sensor component to restrict movement of the sensor component,
the applicator being coupled to the cartridge device to couple the electronic component with the sensor component to form a patch component, as the sensor component moving away from the support mechanism under the action of the electronic component and the applicator, the assist mechanism providing a force to the electronic component away from the support mechanism.

14. The analyte monitoring system according to claim 13, wherein,
the sensor component comprises a connecting base, and a sensor and a sharp object arranged on the connecting base,
the electronic component comprises an outer casing and a receiving portion for receiving the sensor component, the receiving portion comprising an accommodating portion and a through hole longitudinally penetrating the outer casing,
when the electronic component is coupled to the sensor component, the connecting base is embedded in the accommodating portion and the sharp object is at least partially located within the through hole.

15. The analyte monitoring system according to claim 14, wherein,
the applicator comprises a first drive mechanism, a second drive mechanism, a proximal end close to the host and a distal end away from the host during operation, the first drive mechanism is configured to apply a force to the patch component in a manner toward the proximal end, the patch component is driven by the first drive mechanism toward the proximal end to place the sensor at least partially subcutaneously into the host via the sharp object, and after the sharp object reaches a predetermined position, the sharp object is driven by the second drive mechanism toward the distal end to withdraw the sharp object from the host.
